# EUROPEAN PATENT APPLICATION

(11) **EP 1 741 464 A2**
(43) Date of publication of application: **10.01.2007**
(21) Application number: 06014031.6
(22) Date of filing: 06.07.2006
(51) Int. Cl.: A61M 39/24

(54) **One-way valve for an inflatable chamber of a medical device**

(30) Priority: 07.07.2005 US 697255 P
(71) Applicant: Bristol-Myers Squibb Company, New York, N.Y. 10154 (US)
(72) Inventor: Drechsler, Steven, Easton Pennsylvania 18045 (US); Veerapaneni, Bhavani, Princeton New Jersey 08540 (US); Johnsen, Kenneth, Piscataway New Jersey 08854 (US); Lesko, Marc, Jackson New Jersey 08527 (US); Blum, John, Toms River New Jersey 08757 (US); Cline, John, New Brunswick New Jersey 08901 (US)
(74) Representative: Holmes, Miles Keeton

(57) **Abstract**

A medical device such as an ostomy appliance (10) has an inflatable seal (12) for sealing an opening in a patient's body. A non-return valve is provided at an inlet port (22) for admitting inflation fluid into the inflatable seal and for preventing escape of the inflation fluid. The non-return valve comprises a film panel (26) having a first portion (26a) attached to a wall portion (27), and a second portion (26b) that is unattached to the wall portion to provide a valve function. The second portion (26b) lifts from, or seals against, the wall portion (27) in response to a differential fluid pressure acting on the second portion. The wall portion (27) may be a second film panel (28), or an underlying substrate (26).

## Description

### Field of the Invention

The present invention may relate to the field of medical devices, and especially to a one-way valve for an inflatable chamber of a medical device. One aspect of the invention may relate to a valve used in an inflatable seal of an ostomy appliance.

### Background to the Invention

The creation of an ostomy (stoma) is the therapy for many sufferers of diseases or injury of the gastrointestinal or urinary tract. An ostomy is the rerouting of the tract through the abdominal wall to outside the patient's body. Once a stoma has been created, the patient must, usually for the rest of his or her life, use a device worn on the body for capturing or containing the body waste. This has traditionally been done with a bag or pouch attached to the body with adhesive patches or constricting belts. However, the wearing of such a pouch can be an extremely embarrassing and belittling experience for many ostomates. A pouch requires significant changes to a person's public and personal activities.

A controlled evacuation appliance offers the potential for an ostomate to return to some form of normality. The appliance is used to block the stoma mouth, in order to store the liquid and/or solid stool temporarily inside the tract.

For example, US Patent No. 6723079 describes a controlled evacuation device that utilizes an inflatable membrane seal to control the release of effluent from a stoma. The performance and characteristics of the seal may be dependent on the inflation pressure. For such a membrane seal to function properly, a valve is required that allows entry of air into the membrane seal and, once it is filled with air, prevents the release of the air. Should air escape accidentally, the pressure in the membrane seal may become reduced, possibly leading to leakage of effluent, which is likely to be highly embarrassing for the wearer.

Although not relevant to the above field nor technical problem, reference may be made to US Patent No. 5178281 which discloses a check-valve for a flexible hollow-shaped packaging/cushioning balloon. In such an application, the balloon is inflated to provide an air cushion, but the inflation pressure is not particularly important, and limited leakage of air through the inflation valve may be tolerated. The check-valve comprises two films welded in a particular configuration to define a narrowing valve passage. The check-valve is designed to be completely flexible, integral with the balloon, and made of the same flexible material as the balloon, so as to avoid the check valve tearing the balloon material during storage and transportation prior to use.

### Summary of the Invention

In one non-limiting aspect, it would be desirable to provide a non-return (one way) valve that is one or more of: (i) effective to prevent backward flowing of air from the membrane seal, (ii) is reliable in use and will not be affected by body movement; (iii) is sufficiently small to facilitate placement in the appliance, and to avoid increasing significantly the size of appliance; (iv) is light in weight so as not to increase significantly the weight of the appliance that is worn on the body; (v) is inexpensive to manufacture so as not to add undesirably to the cost of the appliance.

Although the invention finds particular application to the field of ostomy appliances, the valve may be used in any medical device where one or more of the above properties may be beneficial.

Broadly speaking, one aspect of the invention may provide a one-way valve for an inflatable chamber of a medical device, the valve comprising a wall portion and a film panel. The wall portion may have an opening therein communicating with an inlet port for inflation fluid. The film panel may have a first portion that is attached to the wall portion for anchoring the film panel, and a second portion that is unattached to the wall portion, and may be movable relative to the wall portion. The second portion may provide a valve seal in response to a differential fluid pressure acting on the film panel.

For example, fluid under pressure in the inflatable chamber may act on one side of the film panel to urge the film panel into sealing engagement with the wall portion.

Fluid pressure at the opening may act on the opposite side of the film panel to urge the film panel to lift from the wall portion. This can provide a simple, yet highly effective and small, one-way valve that is self-closing to avoid escape of fluid pressure in the inflatable chamber, yet is able to open easily when inflation fluid at a higher pressure is injected at the inlet. Another desirable characteristic of this valve is the ability to open or close with a very small pressure differential.

The wall portion may be, or comprise part of, a substrate more rigid and/or substantial than the film panel. For example, the wall portion may be part of a containing wall of, or for, the inflatable chamber. Alternatively, the wall portion may comprise a second film panel. The second film panel may be attached to a substrate having an inlet port therethrough.

The wall portion may be substantially rigid, or it may be flexible. The film panel may be less rigid than the wall portion. The film panel may be of elastic material, or the material may be non-elastic. One or both of the wall portion and the film panel may be of plastics.

The wall portion and/or the substrate may provide structural stability for the valve, thereby to improve the sealing properties of the valve, and provide consistent seal performance.

The film panel and the wall portion may define a fluid flow path therebetween. The fluid flow path may be generally parallel to the wall portion. The fluid flow path may be generally perpendicular to the direction in which fluid enters the valve through the opening.

Further ideas, features and advantages of the invention will be apparent from the following detailed description. Although certain significant features have been described above and/or in the appended claims, the Applicant claims protection for any novel feature or idea described herein and/or illustrated in the drawings, whether or not emphasis has been placed thereon.

### Brief Description of the Drawings

Non-limiting preferred embodiments of the invention are now described, by way of example, with reference to the accompanying claims and drawings, in which:
Fig. 1 is a schematic cross section showing a principle of operation of a first embodiment of valve for an inflatable stoma seal of a controlled evacuation ostomy appliance;
Fig. 2 is a schematic cross section similar to Fig. 1, but showing only the valve and substrate;
Figs. 3 and 4 are schematic perspective views showing assembly of the valve of Figs 1 and 2;
Fig. 5 is a schematic perspective view of an alternative embodiment of valve having a lobed shape;
Fig. 6 is a schematic perspective view of an alternative embodiment of valve having a short lobe shape;
Fig. 7 is a schematic perspective view of an alternative embodiment of valve having a rectangular shape;
Fig. 8 is a schematic perspective view of an alternative embodiment of valve having a rectangular shape;
Fig. 9 is a schematic perspective view of an alternative embodiment of valve having multiple inlet apertures;
Fig. 10 is a schematic exploded view of an alternative embodiment of valve having two film panels, with the bottom panel attached to the substrate around the inlet port, and with both film panels attached to one another to form the fluid flow path through the valve;
Fig. 11 is a schematic side section of the valve of Fig. 10;
Fig. 12 is a schematic perspective view of an alternative embodiment of valve having two fixed film panels, both of which are fixed to the substrate to form the fluid flow path through the valve;
Fig. 13 is a schematic perspective view of an alternative embodiment of valve having film panels that are fixed to the substrate for part of their length and are attached to each other but detached from the substrate for part of their length; and
Fig. 14 is a schematic perspective view of an alternative embodiment of valve having an opening element.

### Detailed Description of Preferred Embodiments

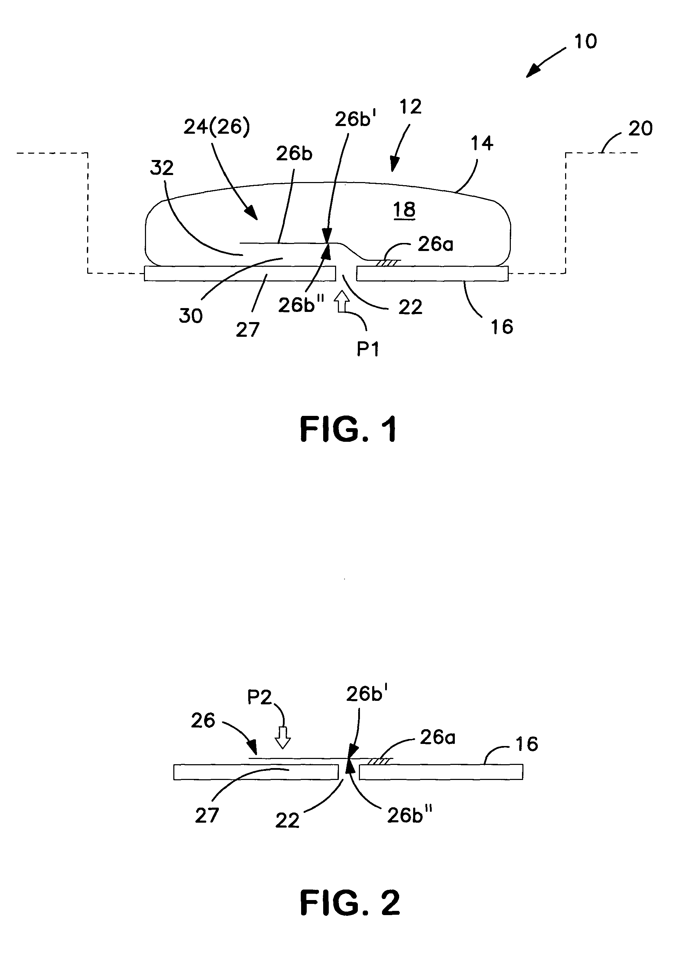

Referring to Figs. 1 to 4, a medical device may take the form of an ostomy appliance 10. The ostomy appliance 10 may comprises an inflatable membrane seal 12 for forming a seal against a person's stoma (not shown) for temporarily blocking the discharge of body waste. The ostomy appliance 10 may be a controlled evacuation appliance for temporary storage of body waste within the stomal tract, until a discharge is desired by the ostomate. The inflatable membrane seal may comprise a flexible plastics membrane wall 14 supported by a substrate 16 to define an inflatable chamber 18. The substrate may act as rear containment wall of, of for, the inflatable chamber 18. The substrate 16 may be directly or indirectly attached to a mounting plate (indicated in phantom at 20) for attachment to the body in the region of the stoma. The substrate 16 may be relatively rigid (at least compared to the film panel 26). The substrate 16 may be made of plastics. The substrate 16 may provide structural and/or dimensional stability for the valve 24. The substrate 16 may comprise a plurality of materials or components attached together. For more details of a controlled evacuation ostomy appliance, reference may be made to the aforementioned US Patent No. 6723079, the content of which is hereby incorporated by reference.

The inflatable chamber 18 may be inflated using any suitable inflation fluid, for example, air, water or saline. The inflation fluid may be injected into the inflatable chamber 18 via an inlet port 22 in the substrate 16. A valve 24 may be disposed inside the inflatable chamber 18 to control the flow of fluid through the inlet. The valve 24 may be a pressure-responsive valve. The valve 24 may be configured as a non-return valve. The valve 24 may open when the pressure of fluid at the inlet port 22 exceeds the pressure within the inflation chamber 18, thereby to admit fluid into the inflatable chamber 18. The valve 24 may close when the pressure of fluid at the inlet port is less than the pressure within the inflatable chamber 18, thereby to preserve the inflation pressure by obstructing escape of the fluid via the inlet port 22.

The valve 24 may comprise a film panel 26 overlying a wall portion 27 around the inlet port 22. In this embodiment, the wall portion 27 may be a portion of the substrate 16. In an alternative embodiment (described later below), the wall portion may comprise a second film panel attached to the substrate 16.

The film panel 26 may comprise a first portion 26a attached to the wall portion 27 to anchor the film panel 26 relative to the wall portion 27. The film panel may further comprise a second portion 26b that is unattached to the wall portion 27 and overlies the inlet port 22. The second portion 26b may have a first face 26b' that faces towards the inflatable chamber 18 (e.g. faces away from the wall portion 27) and is exposed to the internal pressure within the inflatable chamber 18. The internal pressure may act on the first face 26b' in a direction to urge the second portion 26b of the film panel 26 towards the wall portion 27, to close the valve 24. The second portion 26b may have a second face 26b'' that faces towards the wall portion 27 and is exposed to the pressure at the inlet port 22. The pressure at the inlet port 22 may act on the second face 26b'' in a direction to urge the second portion 26b away from the wall portion 27, to open the valve 24. The position adopted by the second portion 26b may therefore depend on a differential between the internal pressure and the pressure at the inlet port 22.

Referring to Fig. 1, in use, when the fluid pressure at the inlet port 22 exceeds the internal pressure, the pressure differential P1, acting on the second face 26b" causes the second portion 26b to lift away from the wall portion 27, opening a gas flow path 30 from the inlet port 22 to a valve exit 32 communicating with the inflatable chamber 18. Gas is thereby admitted through the valve 24 into the inflatable chamber 18.

Referring to Fig. 2, when the fluid injection pressure is removed from the inlet port 22, and the internal pressure exceeds the pressure at the inlet port 22, the pressure differential P2 acting on the first face 26b' causes the second portion 26b to be pressed against the wall portion 22. The second portion 26b may thus seal the inlet port 22 to prevent escape of inflation fluid.

The valve 24 may have a simple construction and be very light in weight with very few parts, but may still provide a reliable self-closing valve action to block unwanted deflation of the inflatable chamber 18. The internal pressure within the inflatable chamber 18 is exploited to provide a sealing force for closing and sealing the valve 24. The substrate 16 may provide structural and/or dimensional stability for the valve 24, which may improve the valve performance and/or enable consistency amongst different valves. Moreover, the valve 24 may have a very low profile, such that it does not occupy a substantial volume, nor does it increase undesirably the height of the inflatable chamber 18 with respect to the substrate 16. The gas flow path 30 through the valve 24 may be in a direction generally parallel to the wall portion 27 and/or the substrate 16. The gas flow path 30 may be generally perpendicular to the direction of gas flow through the inlet port 22.

The attachment between the first portion 26a of the film panel 26 and the wall portion 27 may be achieved by any suitable means. For example, the attachment may be a weld (formed for example by heat welding, ultrasonic welding, laser welding or radio frequency welding), a solvent or chemical bond, an adhesive bond, or it may be formed by integral molding (for example, insert molding, multi-shot injection molding, or insert thermoforming). The first portion 26a may be shaped as one or more continuous or discontinuous line segments. The first portion 26a may extend around at least a portion (or at least a majority) of a periphery of the film panel 26, leaving at least one unsecured peripheral edge portion to form the valve exit 32. Alternatively, the valve exit 32 may be defined by an aperture (not shown) in the film panel 26, and the first portion 26a may define a closed loop shape, for example, around the entire periphery of the film panel 26.

The shape of the film panel 26, and the configuration of the first portion 26a, may take any desired form. In the embodiment of Figs. 1-4, the film panel 26 may have a so-called "keyhole" shape, including a round portion from which projects a lateral extension or chute. The first portion 26a may extend around substantially the entire periphery except for an unsecured region at the extremity 26c of the lateral extension, to define the valve exit 32.

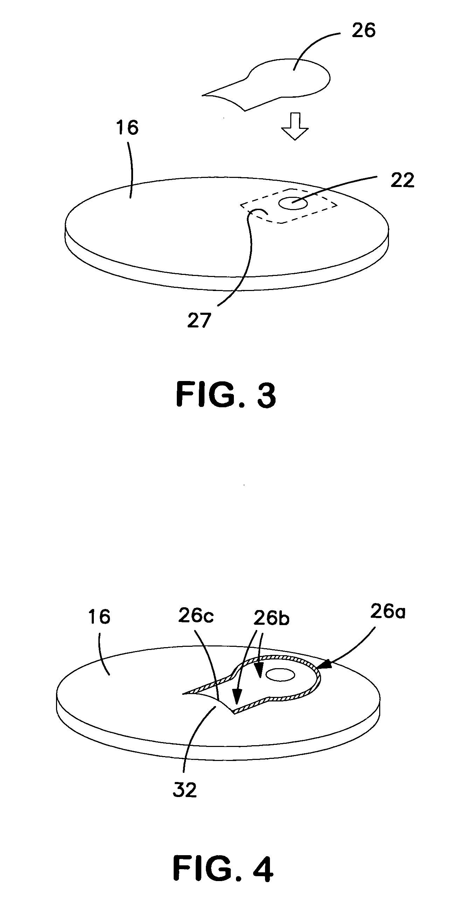

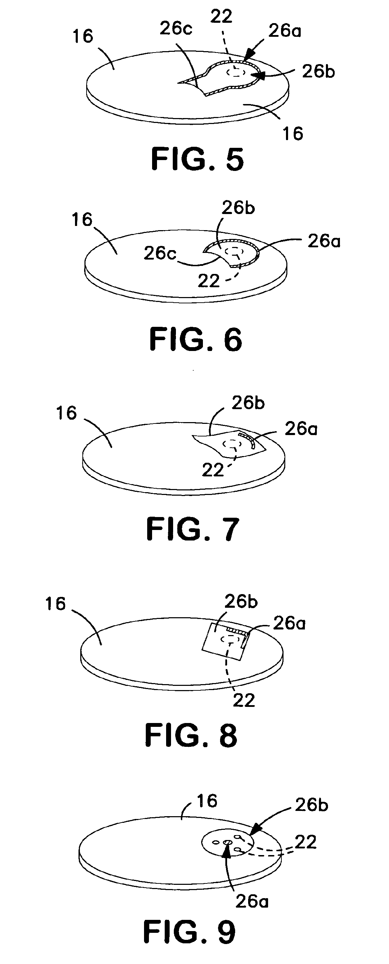

In an alternative embodiment of Fig. 5, the film panel 26 may have an elongated lobe shape, instead of a key-hole shape. The first portion 26a may extend around a majority of the periphery (e.g. the smoothly curved portion), leaving the extremity 26c unsecured, similarly to that illustrated in Fig. 4.

In an alternative embodiment of Fig. 6, the film panel 26 may have a shorter lobe shape. The first portion 26a may extend around a majority of the periphery (e.g. the smoothly curbed portion), leaving a flat 26c unsecured, similarly to that illustrated in Fig. 5.

In an alternative embodiment of Fig. 7, the film panel 26 may have a generally rectangular shape. The first portion 26a may extend over a short distance close to the inlet port 22. The first portion 26a may be linear or it may be arcuate. In this embodiment, the majority of the periphery of the film panel 26 may be unsecured, to define a large exit 32. Nevertheless, the first portion 26a may still perform the desired function of anchoring the film panel 26 with respect to the wall portion 27, and the valve 24 may function in the same manner as described previously.

In an alternative embodiment of Fig. 8, the film panel may again have a generally rectangular shape. The first portion 26a may extend at one or more corners of the rectangular shape, leaving the majority of the periphery of the film panel 26 unsecured.

In an alternative embodiment of Fig. 9, the inlet port 22 may be configured as plural apertures (e.g., 3 apertures) in the substrate 16. The film panel 26 may be shaped and dimensioned to cover all of the apertures. The film panel 26 may, for example, be circular. The first portion 26a may be configured to be between two or more of the apertures. In the present embodiment, the first portion 26a may be near a centre of the film panel 26 and/or near a central point with respect to the apertures.

In the foregoing embodiments, the wall portion 27 may be provided as a portion of the substrate 16. In these embodiments, the seal performance may be dependent on the seal properties between the film panel 26 and the surface of the substrate 16. In an alternative form, the wall portion 27 may comprise a second film panel between the film panel 26 and the substrate. The seal performance may thus be dependent on the seal properties between the two film panel 26 and 28, which may provide alternative characteristics or design options for a designer.

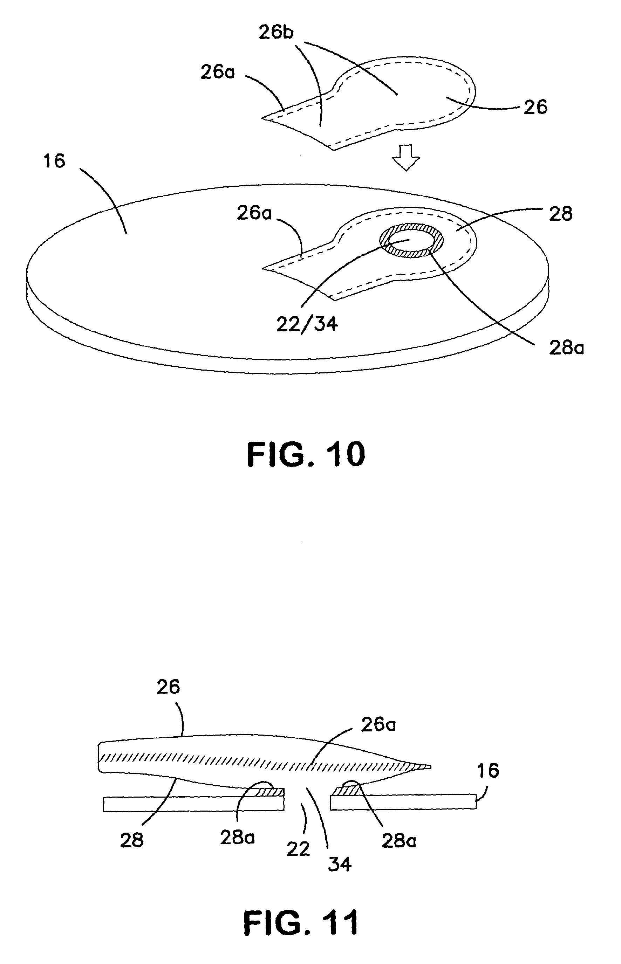

Figs. 10 and 11 may illustrate an embodiment including a second film panel 28 to provide the wall portion 27. The second film panel 28 may comprise an inlet aperture 34 in register with the inlet port 22 for admitting inflation fluid from the inlet port 22 into the space between the two film panels 26 and 28. The second film panel 28 may be attached to the substrate at a third portion 28a. The third portion 28a may be of closed loop form, and may encircle the inlet aperture 34. In the embodiment illustrated in Figs. 8 and 9, the third portion 28a may be limited to being adjacent to the inlet aperture 34, such that a majority of the second film panel 28 may be unsecured to the substrate 16. The first region 26a of the first film panel 26 may define the region of attachment between the two film panels 26 and 28 (e.g. along a majority of a peripheral edge). The two film panels 26 and 28 may thus define a pouch-like valve structure that is anchored to the substrate 16 at the inlet port 22. The seal properties may be influenced by the fact that the first film panel 26 may no longer be anchored relative to the substrate 16, but may float to some extent when the pressure at the inlet port 22 is sufficient to inflate the valve 24 to admit fluid into the inflatable chamber 18.

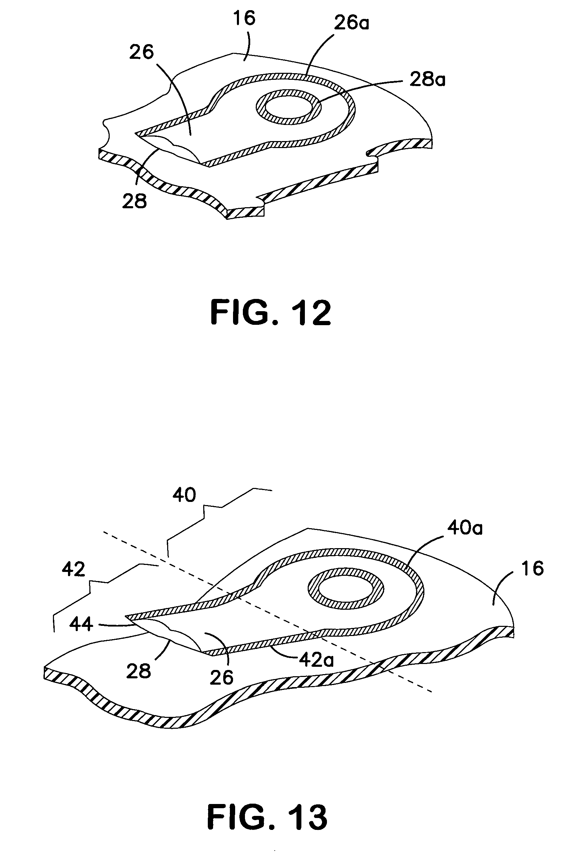

Fig. 12 may illustrate a slight modification of the embodiment of Figs. 10 and 11, in which the second film panel 28 is attached to the substrate 16 at the third portion 28a and additionally at the first portion 26a. In other words, the first portion 26a defines a line of mutual attachment of the sandwich consisting of first film panel 26, the second film panel 28 and the substrate 16. Such an implementation may anchor the first film panel 26 more securely (as in the first embodiment), but also provide the seal performance associated with having two film panels.

Figs. 13 may illustrate a hybrid of the embodiments of Figs. 10-12, in which the attachment between the first and second film panels 26 and 28 is configured in two zones 40 and 42. In a first zone 40 (e.g., the curved edges of the film panels), the two film panels 26 and 28 are attached to each other and also to the substrate 16, along line 40a to anchor both film panels 26 and 28 to the substrate 16. In a second zone 42, (e.g. the straight edges of the lateral extension 44), the two film panels 26 and 28 may be attached to each other along lines 42a, but not to the substrate 16. The lateral extension 44 may float relative to the substrate 16.

The seal properties of the valve 24 (e.g. as shown in any of the foregoing embodiments) may be adjusted or enhanced by one or more of the following techniques:
(a) Adjusting the thickness and/or stiffness of the film panel(s) 26 (and 28). For example, the thickness of the film may be in the range of from about 0.01mm to 2mm, depending on the desired characteristics. It is also possible to alter the valve characteristics by means of a film panel or substrate with more than one thickness, for example, by means of extrusion or fabrication.
(b) Selection of the constituent materials for the film panel(s) 26 (and 28) and/or the substrate 16. For example, suitable materials may include, but are not limited to: silicone rubber; ethylene vinyl acetate; polyethylene; and ABS. It is also possible to create a film panel or substrate with more than one material, by means of multi-material processes, such as multi-shot injection or co-extrusion.
(c) Adjusting the surface properties of the film panel(s) 26 (and 28) and/or the substrate 26. For example, a highly polished surface or a matte finished surface, or combinations of these two extremes, may be appropriate for specific applications. It may also be desirable to vary the surface properties of the valve film or substrate materials either locally or over the entire surface of the valve. Such surface treatments may, for example, include corona treatment, or surface treatments that render the surfaces hydrophobic or hydrophilic.
(d) Including topographical features in the film panel(s) 26 (and 28) and/or the substrate 16. Such features may, for example, include one or more of: localized dimples; raised bumps; grooves; ridges; corrugations; and pebbled surface features. The features may be arranged randomly or in a repeating pattern. The features may range in size from about 0.02mm to about 5mm across (e.g. diameter), for example, from about 0.02mm to about 2mm. Additionally or alternatively, the features may range in size from about 0.02mm to about 5mm in height or depth, for example, from about 0.02mm to about 2mm. In the case or grooves, corrugations or ridges provided in either a film panel or the substrate 16, such features may be aligned either parallel to, or perpendicular to, the direction of flow of fluid through the valve 24, in order to control the valve properties. The grooves, corrugations or ridges may have a length in the range of from about 5% to 100% of the respective dimension (length or width) of the valve 24. Alternatively, the grooves, corrugations or ridges may have a mixed or random orientation.
(e) Introducing a substance such as a liquid medium into the valve 24. The substance may be introduced during manufacture, after manufacture, or just prior to use of the medical device 10. The substance may, for example, be water, oil, wax or any other medium that may alter the flow through the valve, improve its sealing ability or enhance its stability over an extended shelf life.

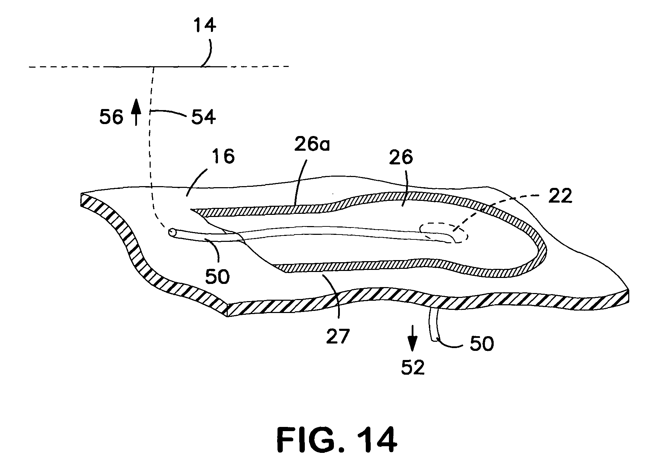

Referring to Fig. 14, an opening element 50 may be incorporated into the valve 24. The opening element 50 may serve to hold the valve 24 at least partly open after manufacture and up until the time the medical device 10 is to be employed for use. The opening element 50 may allow pressure equalization between the inflatable chamber 18 and the ambient surroundings, to ensure that any fluid (e.g. residual air) in the inflatable chamber does not become trapped and cause premature inflation if, for example, the ambient pressure is reduced (for example, during transportation by air).

The opening element 50 may extend through the inlet port 22 and partly, locally separate the film panel 26 from the wall portion 27. Just prior to use of the medical device 10, the opening element 50 may be extracted via the inlet port 22 by pulling in the direction of arrow 52. Removal of the opening element 50 allows proper operation of the valve 24. The opening element may, for example, comprise a filament. The filament may be of woven thread or string, or a polymer monofilament, or a length of polymer film. The cross-section dimension of the filament may range from about 0.02mm to about 2mm. As an alternative to manual extraction of the opening element 50, the opening element 50 may be coupled to a moving portion of the inflatable membrane seal 12 (for example, to the membrane 14 as indicated in phantom at 54) and configured such that, when the seal 12 is inflated the first time, the act of inflation pulls the opening element 50 in the direction of arrow 56 to withdraw the opening element 50 internally from the valve 24.

It will be appreciated that any of the above described embodiments may be combined together, to combine their respective features and characteristics.

Although the preferred embodiments have been described in relation to an inflatable seal of an ostomy appliance, it will be appreciated that the principles of the valve of the present invention may be used in any medical device, and especially a medical device employing an inflatable seal.

The foregoing description is merely illustrative of preferred forms of the invention. Many modifications, improvements and/or equivalents may be used without departing from the scope and/or spirit of the invention.

## Claims

1. A medical device comprising an inflatable chamber, an inlet port for inflation fluid, and a one-way valve located in the chamber and communicating with the inlet port for controlling the flow of inflation fluid through the inlet port, the valve comprising:
a wall portion having an opening therein communicating with the inlet port; and
a film panel comprising
a first portion attached to the wall portion for anchoring the film panel, and
a second portion that
(i) overlaps the inlet port and at least a part of the wall portion,
(ii) is unattached to the wall portion so as to be displaceable relative to the wall portion in response to a differential fluid pressure between the pressure in the inflatable chamber and the pressure at the inlet port,
(iii) has a chamber facing surface that is exposed to fluid pressure in the inflatable chamber such that said fluid pressure in the inflatable chamber urges the second portion of the film panel into sealing contact with the wall portion to obstruct escape of inflation fluid from the inflatable chamber, and
(iv) has an inlet facing surface that is exposed to fluid pressure at the inlet port such that said fluid pressure at the inlet port urges the second portion of the film panel to lift away from the wall portion for admitting inflation fluid through the inlet port.

2. The medical device according to claim 1, wherein, in use, the valve adopts a position that is dependent on a pressure differential between the pressure at the inlet and the pressure in the internal chamber.

3. The medical device according to claim 1 or 2, wherein the device is an ostomy appliance.

4. The medical device according to claim 1, 2 or 3, wherein the device comprises an inflatable seal for sealing an opening in a human body, the inflatable seal comprising the inflatable chamber.

5. The medical device according to any preceding claim, wherein the wall portion comprises a second film panel.

6. The medical device according to claim 5, wherein the second film panel is attached to a substrate more rigid than the second film panel.

7. The medical device according to any preceding claim, wherein the wall portion is substantially rigid.

8. The medical device according to nay of claims 1 to 6, wherein the wall portion is flexible.

9. The medical device according to any preceding claim, wherein the film panel has a thickness in the range of from about 0.01 mm to about 2mm.

10. The medical device according to any preceding claim, wherein the film panel and the wall portion define a direction of fluid flow through the valve that is generally parallel to the wall portion.

11. The medical device according to any preceding claim, wherein the film panel and the wall portion define a direction of fluid flow through the valve that is generally perpendicular to a direction of entry of fluid through the opening in the wall portion.

12. The medical device according to any preceding claim, wherein an area of the second portion of the film panel is at least twice the size of a cross-sectional area of the opening in the wall portion.

13. The medical device according to any preceding claim, wherein the first portion is shaped as one or more segments of a line.

14. The medical device according to any preceding claim, wherein first portion is attached to the wall portion by one or more selected from: a weld; an adhesive; a solvent or chemical bond; or an integral plastics molding.

15. The medical device according to claim 14, wherein the weld is selected from: heat welding; ultrasonic welding; radio frequency welding; a solvent or chemical bond; or laser welding.

16. The medical device according to claim 14, wherein the integral plastics molding is selected from: insert molding; multi-shot injection molding; and insert thermoforming.

17. The medical device according to any preceding claim, wherein at least a portion of the film panel is substantially rigid or stiff.

18. The medical device according to any preceding claim, wherein at least a portion of the film panel is flexible.

19. The medical device according to any preceding claim, wherein the film panel comprises at least one relatively rigid or stiff portion and at least one flexible portion to allow localized valve movement.

20. The medical device according to any preceding claim, wherein at least one selected from the film panel and the wall portion has a first portion with a first thickness and a second portion with a second thickness.

21. The medical device according to any preceding claim, wherein the substrate comprises a plurality of materials or components attached together.

22. The medical device according to claim 21, wherein the plurality of materials or components are attached together by at least one selected from: welding; adhesive; solvent or chemical bond; integral plastics molding.

23. The medical device according to claim 22, wherein the welding is selected from: heat welding; ultrasonic welding; laser welding; or solvent or chemical bonding.

24. The medical device according to claim 22, wherein the integral plastics molding is selected from: insert molding; multi-shot injection molding; and insert thermoforming.

25. The medical device according to any preceding claim, wherein at least a portion of the film panel and/or a portion of the wall portion has a highly polished surface.

26. The medical device according to any preceding claim, wherein at least a portion of the film panel and/or a portion of the wall portion has a matte surface finish.

27. The medical device according to any preceding claim, wherein at least a portion of the film panel and/or a portion of the wall portion has a non-planar topographical surface characteristic.

28. The medical device according to claim 27, wherein the non-planar topographical surface characteristic comprises one or more selected from: bumps; dimples; ridges; grooves; and corrugations.

29. The medical device according to claim 27 or 28, wherein the non-planar topographical surface characteristic is aligned generally randomly.

30. The medical device according to claim 27 or 28, wherein the non-planar topographical surface characteristic is aligned generally parallel to a direction of fluid flow through the valve.

31. The medical device according to claim 27 or 28, wherein the non-planar topographical surface characteristic is aligned perpendicular to a direction of fluid flow through the valve.

32. The medical device according to any preceding claim, wherein a substance is applied to one or more of the confronting surfaces of the film panel and the wall portion.

33. The medical device according to claim 32, wherein the substance is selected from: oil; water; saline; and wax.

34. The medical device according to any preceding claim, wherein at least a portion of a confronting surface of the film panel and/or the wall portion is modified to alter the surface characteristics.

35. The medical device according to claim 34, wherein said surface treatment is selected from: corona treatment; hydrophobic treatment; and hydrophilic treatment.

36. The medical device according to any preceding claim, further comprising an opening element positioned removably between the film panel and the wall portion, so as to prevent full closing of the valve.

37. The medical device according to claim 36, wherein the opening element comprises a filament.

38. The medical device according to claim 36 or 37, wherein the opening element projects outwardly through the inlet port, and is removable by extracting the opening element through the inlet port.

39. The medical device according to claim 36 or 37, wherein the opening element is attached to a movable part of the medical device, such that in use, the opening element is removed from the valve when the inflatable chamber is inflated.

40. A one-way valve for an inflatable seal of a medical device, the one-way valve comprising:
an inlet port for inflation fluid;
a wall portion having an opening therein communicating with the inlet port; and
a film panel comprising
a first portion attached to the wall portion for anchoring the film panel, and
a second portion that
(i) overlaps the inlet port and at least a part of the wall portion,
(ii) is unattached to the wall portion so as to be displaceable relative to the wall portion in response to a differential fluid pressure between the pressure in the inflatable seal and the pressure at the inlet port,
(iii) has a seal facing surface that is exposed to fluid pressure in the inflatable seal such that said fluid pressure in the inflatable seal urges the second portion of the film panel into sealing contact with the wall portion to obstruct escape of inflation fluid from the inflatable seal, and
(iv) has an inlet facing surface that is exposed to fluid pressure at the inlet port such that said fluid pressure at the inlet port urges the second portion of the film panel to lift away from the wall portion for admitting inflation fluid into the inflatable seal.

41. An inflatable seal for an ostomy appliance, the inflatable seal comprising:
a substrate;
an inflatable membrane supported by the substrate and defining an inflatable chamber, the inflatable membrane having a stoma engaging surface for engaging a stoma to obstruct stomal discharge when the inflatable membrane is inflated in use;
an inlet port in the substrate; and
a one-way valve located in the chamber and communicating with the inlet port for controlling the flow of inflation fluid through the inlet port, the valve comprising:
a wall portion having an opening therein communicating with the inlet port; and
a film panel comprising
a first portion attached to the wall portion for anchoring the film panel, and
a second portion that
(i) overlaps the inlet port and at least a part of the wall portion,
(ii) is unattached to the wall portion so as to be displaceable relative to the wall portion in response to a differential fluid pressure between the pressure in the inflatable chamber and the pressure at the inlet port,
(iii) has a chamber facing surface that is exposed to fluid pressure in the inflatable chamber such that said fluid pressure in the inflatable chamber urges the second portion of the film panel into sealing contact with the wall portion to obstruct escape of inflation fluid from the inflatable chamber, and
(iv) has an inlet facing surface that is exposed to fluid pressure at the inlet port such that said fluid pressure at the inlet port urges the second portion of the film panel to lift away from the wall portion for admitting inflation fluid through the inlet port.

42. The seal according to claim 41, wherein the wall portion is a portion of the substrate.

43. The seal according to claim 41 or 42, wherein the wall portion comprises a second film panel attached to the substrate.
